# FASCICULE DE BREVET EUROPEEN

(11) **EP 1 087 708 B1**
(45) Date de publication et mention de la délivrance du brevet: **07.09.2005**
(21) Numéro de dépôt: 99925126.7
(22) Date de dépôt: 17.06.1999
(51) Int. Cl.: A61B 17/32, A61M 1/00

(54) **PROCEDE DE GENERATION D'UN TRAIN IMPULSIONNEL D'UN JET LIQUIDE STERILE POUR DES APPLICATIONS MEDICALES**
VERFAHREN ZUR ERZEUGUNG EINER IMPULSFOLGE EINES STERILEN FLÜSSIGKEITSSTRAHLES FÜR MEDIZINISCHE ZWECKE
METHOD FOR GENERATING A PULSE TRAIN OF STERILE LIQUID JET FOR MEDICAL USES

(30) Priorité: 19.06.1998 FR 9807879; 22.03.1999 FR 9903630
(43) Date de publication de la demande: 04.04.2001
(73) Titulaire: Eschmann Holdings Limited, West Sussex BN15 8TJ (GB)
(72) Inventeur: GONON, Bertrand, F-69002 Lyon (FR)
(74) Mandataire: Metz, Paul
(86) Numéro de dépôt international: PCT/FR1999/001462
(87) Numéro de publication internationale: WO 1999/065408

(56) Documents cités:
- EP-A- 0 489 496
- DE-A- 3 715 418
- US-A- 3 930 505
- US-A- 4 655 197
- US-A- 5 674 226

## Description

L'invention se rapporte à un procédé de génération d'un train impulsionnel d'un jet liquide stérile pulsé-aspiré et au jet liquide pulsé-aspiré ainsi généré pour l'alimentation d'une pièce à main ou d'un cathéter en vue d'applications notamment chirurgicales et médicales.

On connaît déjà l'utilisation des jets pulsés d'un liquide stérile sous haute pression à des fins d'interventions chirurgicales.

On peut citer par exemple à cet effet EP n° 0636345 au nom de SENTINEL MEDICAL qui concerne un instrument chirurgical à jet pulsé de liquide à des fins de découpe et d'émulsification avec une aspiration conjointe pour l'évacuation du liquide et des résidus biologiques.

Le jet pulsé provient du mouvement de va-et-vient répété d'un piston amplificateur qui reçoit le liquide sous faible pression.

L'inconvénient majeur de ce système concerne l'utilisation d'un piston qui ne peut produire qu'un seul train d'impulsions pendant son trajet de travail le long de sa course. Il convient ensuite de remplir à nouveau la chambre du piston, ce qui condamne ce dispositif à un fonctionnement discontinu peu compatible avec les exigences de travail des chirurgiens.

Il existe également des inventions se rapportant à la chirurgie de l'oeil dans lesquelles un jet pulsé de liquide est dirigé sur l'oeil et notamment sur la cornée en vue d'un travail de désintégration des tissus défectueux et de décollement des matières et corps déposés ou incrustés. Ces inventions sont protégées par les brevets américains n° 3,818,913 et 3,930,505 au nom de WALLACH.

Il s'agit de jets pulsés à haute fréquence destinés par la haute cadence de répétition à un travail de désintégration par trains de frappes pour le nettoyage du cristallin en le débarrassant des tissus défectueux et des matières et corps étrangers. Il est prévu classiquement une aspiration séparée pour l'évacuation du liquide et des résidus du travail de désintégration.

Dans ces derniers dispositifs, l'aspiration est également continue et séparée. Par contre, il ne s'agit pas de découpe mais de décollement et de désintégration en vue de l'évacuation des matières et des tissus gênants.

De façon générale, les jets liquides pulsés à haute pression connus antérieurement sont des trains d'impulsions liquides déclenchés sur commande et projetés sur la zone de dissection.

Le liquide projeté est ensuite évacué par aspiration en continu ou lorsqu'il dépasse une certaine quantité jugée gênante pour la poursuite du travail de dissection.

Dans ces réalisations, le chirurgien ne peut approcher de trop près le tissu à disséquer par l'extrémité de la pièce à main en raison des projections latérales de liquide et des éclaboussures ainsi générées troublant la visibilité du champ opératoire.

Par ailleurs, le rendement de dissection n'est que faiblement meilleur à celui d'un jet continu en raison des phénomènes de rebond.

Finalement, la pénétration de liquide même stérile dans l'ouverture découpée et ceci en quantités faibles mais non négligeables, est un inconvénient pour le corps du patient qui doit l'éliminer en plus de toutes les autres surcharges liées à l'intervention.

DE-A-37 15 418 montre un procédé tel que défini dans la partie introductive de la revendication 1.

La présente invention a pour but de remédier aux inconvénients précités en proposant un procédé de génération d'un train impulsionnel d'un jet liquide stérile pulsé-aspiré pour l'alimentation d'une pièce à main, d'un cathéter ou analogue, en vue d'applications chirurgicales ou médicales. Le procédé selon l'invention est défini par la revendication 1. Les revendications dépendantes concernent des variantes du procédé selon l'invention. Ce procédé permet de créer un jet éjecté et aspiré d'une grande efficacité opératoire.

En outre l'invention concerne un circuit selon la revendication 25 pour la mise en oeuvre procédé selon l'invention. Ce circuit se caractérise en ce que l'on met en oeuvre une voie de liquide sous pression par exemple sous haute pression et une voie pneumatique d'aspiration et en ce que l'on commande périodiquement le tir du jet sous pression pendant l'application périodique de l'aspiration et en ce qu'on libère le tissu pendant la coupure de l'aspiration par une mise à l'air.

Le procédé selon l'invention présente de nombreux avantages.

Il permet d'éviter les projections latérales et les éclaboussures de toute sorte et de ce fait procure une bonne visibilité du champ opératoire en même temps que la possibilité d'approcher de très près le tissu à découper.

S'agissant d'un jet pulsé-aspiré, c'est-à-dire d'un jet tiré en même temps que fonctionne l'aspiration, le tissu reste plaqué c'est-à-dire tendu momentanément avant et pendant le tir à l'extrémité du manchon d'extrémité de la pièce à main, puis se détend pendant la phase finale de l'aspiration. On procède ainsi à un tir sur un tissu tendu qui assure précision et propreté de la découpe et du champ opératoire.

La durée d'application du jet étant courte, une faible consommation en liquide stérile est assurée.

Lors de phases particulières du travail chirurgical, ou pour des applications spécifiques, il est souhaitable d'augmenter la fréquence de récurrence du jet de pression.

Or, lorsque la fréquence de récurrence du jet augmente, et/ou lorsque à partir d'une certaine longueur, l'inertie, l'élasticité et l'effet réservoir liés à la déformation du tube d'alimentation de la pièce à main et la pression résiduelle deviennent sensibles, ce procédé de base devient moins performant.

En effet, malgré la présence d'une armature de renforcement, le tube d'alimentation du liquide en haute pression se déforme légèrement. Le front de descente de l'impulsion de pression non seulement s'incline, mais s'arrondit laissant apparaître une zone d'inefficacité augmentant avec la fréquence des impulsions qui empiète sur la zone de récupération et d'aspiration, si bien qu'à partir d'une limite supérieure de fréquences on se rapproche du régime continu en perdant les avantages liés au régime pulsé.

Or, l'existence d'un front de coupure de la haute pression franc, raide et de courte durée est une condition importante de précision, de commodité et d'efficacité dans le travail chirurgical.

La variante du procédé de base a pour but de remédier à ces inconvénients.

Le principe inventif de cette variante consiste à obtenir un front de descente des créneaux de pression du jet de liquide non plus seulement par la coupure de pression et donc celle du jet, mais par une mise en communication simultanée ou quasi simultanée du conduit d'alimentation en liquide avec l'extérieur notamment à l'air ou avec l'aspiration ou le générateur de vide.

Selon la variante du procédé de base, on prévoit, en dérivation sur le tube d'alimentation en liquide sous pression, une branche de conduit reliée au générateur de dépression ou à l'air à travers un organe de coupure-ouverture commandé à la fréquence de récurrence en synchronisme avec les autres commandes, de manière à réaliser, avant le tir suivant, la décharge de la pression résiduelle existant dans le conduit d'alimentation en liquide sous pression. Cette commande périodique de mise en communication du conduit de pression avec l'extérieur s'effectue simultanément ou immédiatement avant ou après la commande de coupure du conduit d'alimentation en haute pression.

Cette variante du procédé présente divers avantages supplémentaires :
. front arrière de l'impulsion de pression marquant l'arrêt bien raide lorsque la fréquence de récurrence augmente ;
. efficacité et propreté dans le travail chirurgical ;
. perfectionnement très facile à mettre en oeuvre.

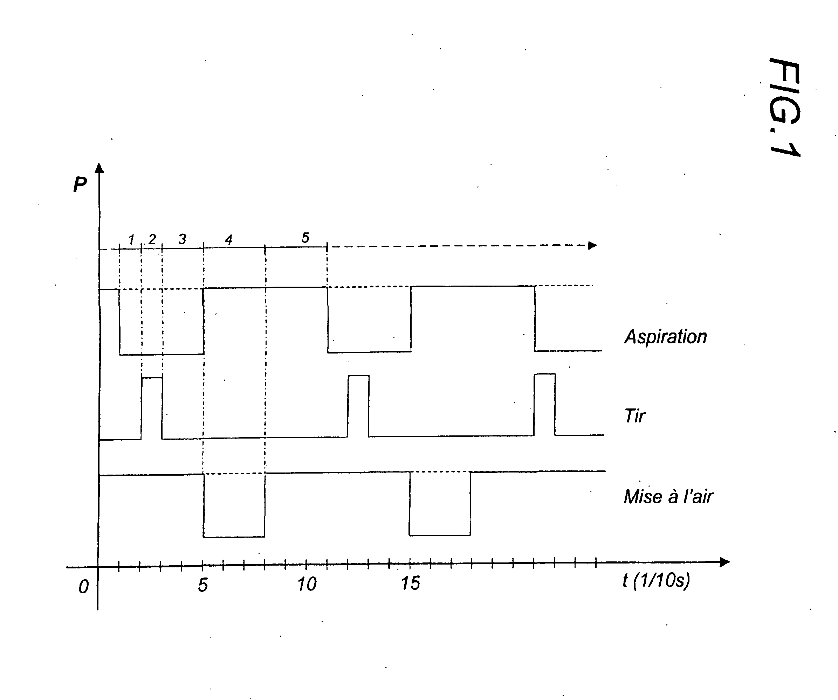

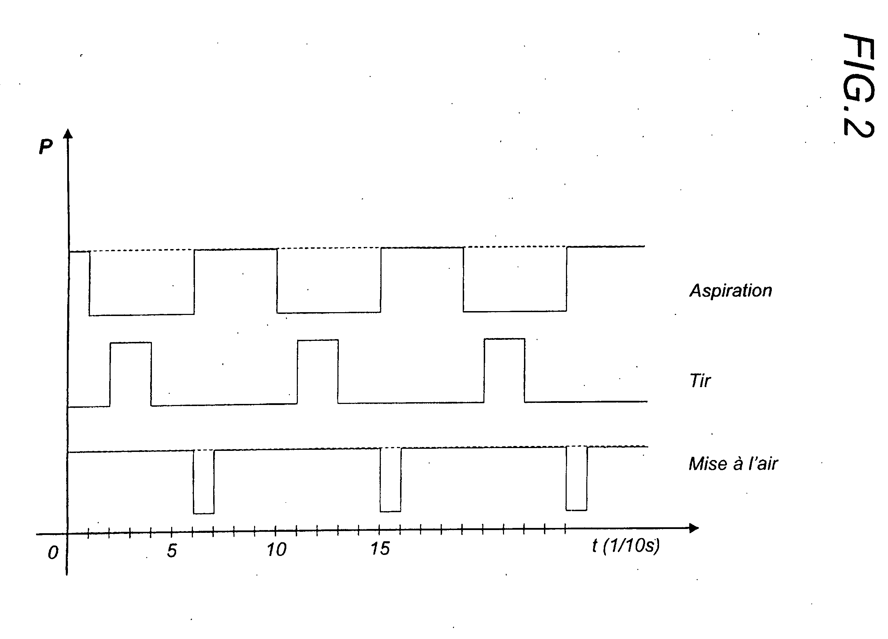

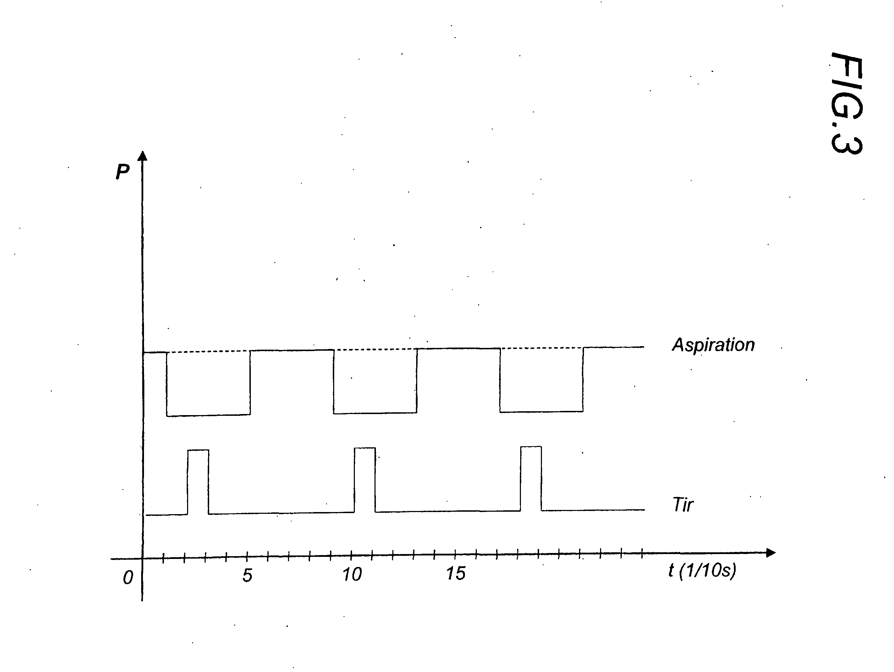

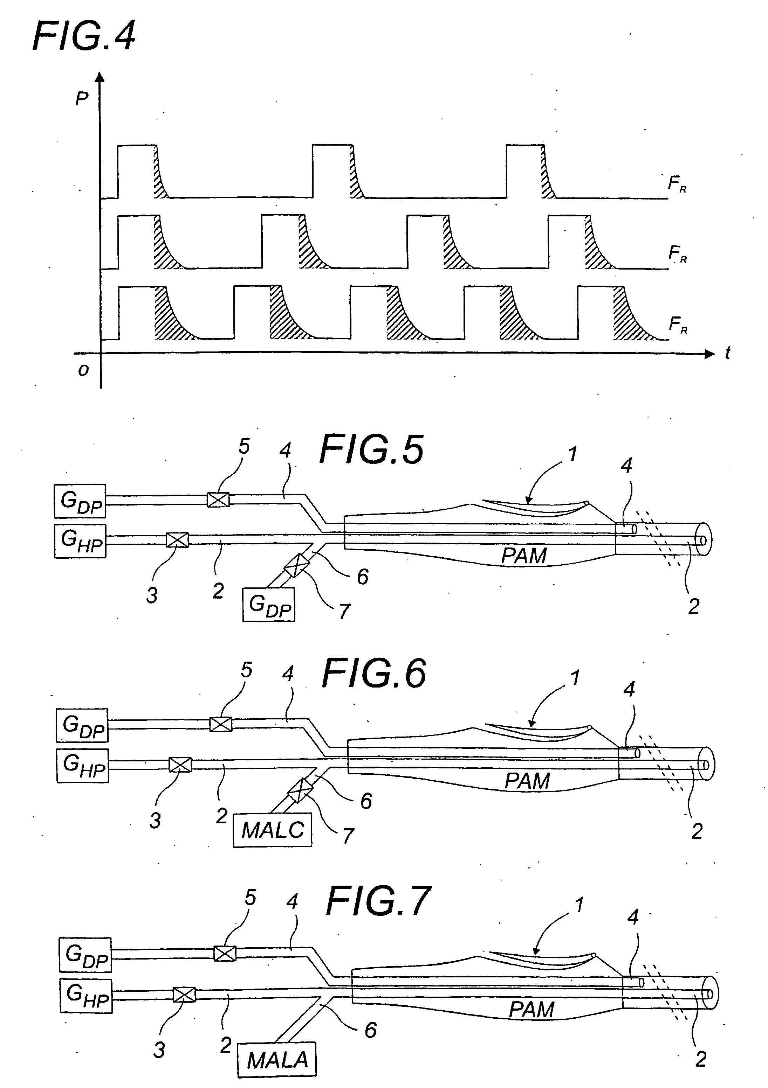

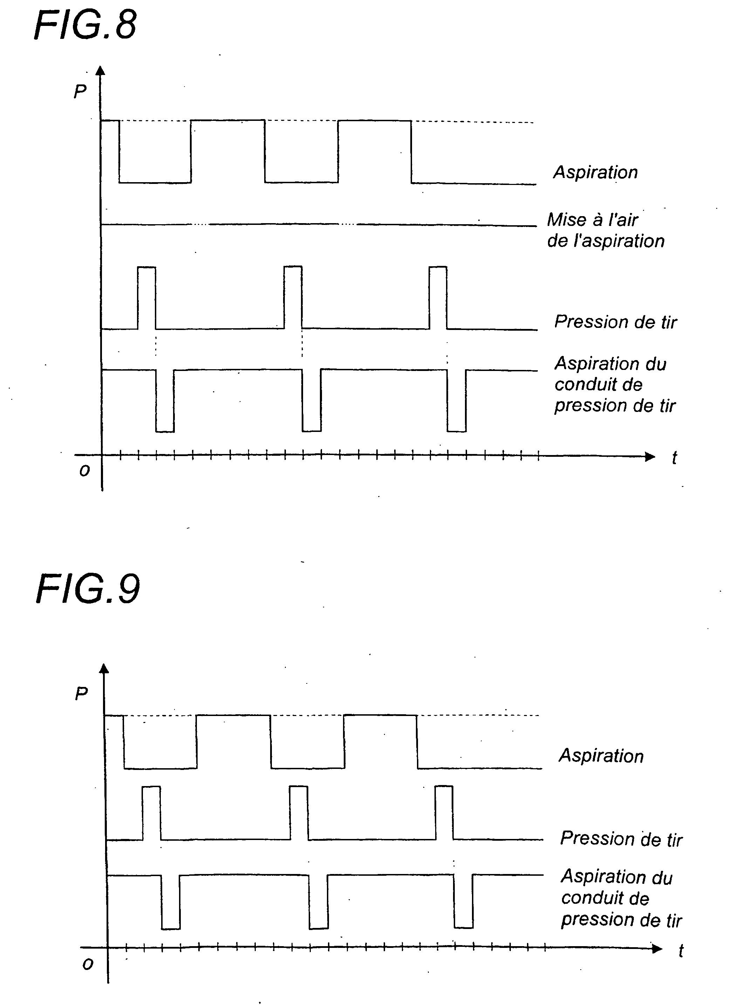

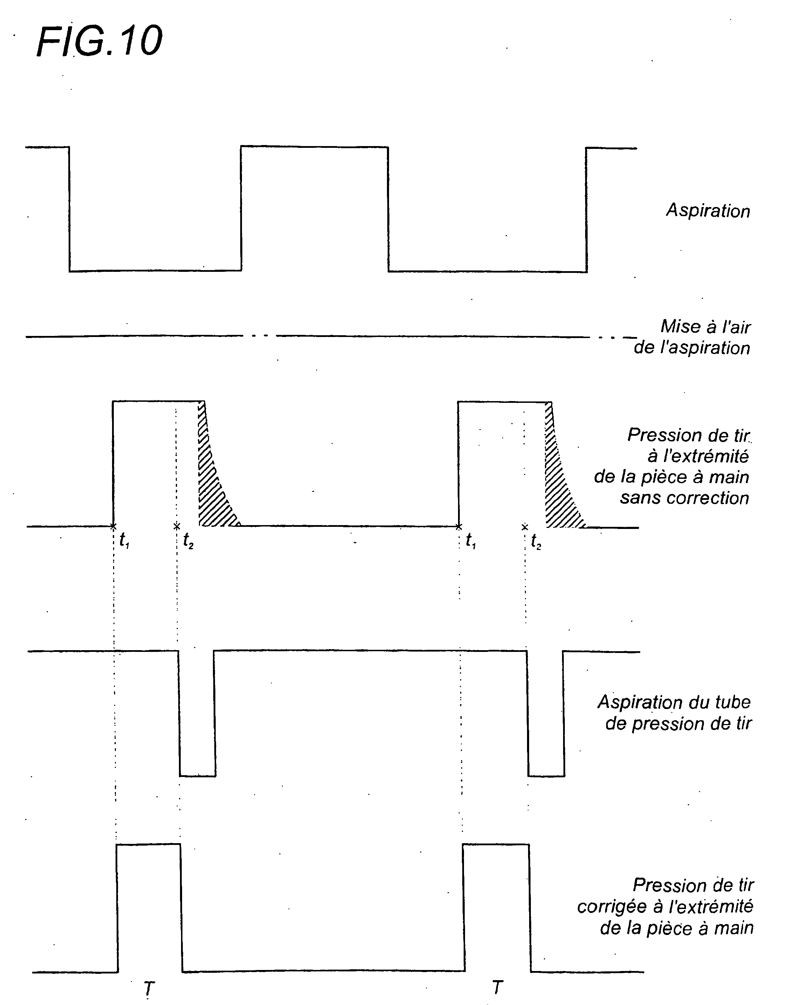

D'autres caractéristiques et avantages de l'invention apparaîtront dans la description qui suit, donnée à titre d'exemple non limitatif en référence aux dessins dans lesquels :
. la figure 1 est un graphique des formes d'ondes de base des paramètres du train impulsionnel en fonction du temps ;
. la figure 2 est un graphique des formes d'ondes selon une première variante à durée de tir plus importante ;
. la figure 3 est un graphique des formes d'ondes de tirs ne faisant pas intervenir de mise à l'air ;
. la figure 4 est une représentation graphique comparative de trois trains d'impulsions de pression P de liquide en fonction du temps « t » à fréquences de récurrence F_{R} croissantes montrant la zone d'inefficacité du procédé de base grandissant avec l'augmentation de la fréquence de récurrence ;
. la figure 5 est une vue du montage de base montrant les liaisons avec les différentes sources et les emplacements des organes de coupure-ouverture en utilisant un générateur de dépression GDP ;
. les figures 6 et 7 sont des vues analogues mais avec une liaison à un dispositif de mise à l'air commandé MALC ou automatique MALA ;
. la figure 8 est un graphique des formes d'ondes des différentes grandeurs en pression P formant le jet pulsé représentées en fonction du temps « t » avec mise périodique à l'air ;
. la figure 9 est un graphique des formes d'ondes des différentes grandeurs en pression P formant le jet pulsé représentées en fonction du temps « t » sans mise périodique à l'air ;
. la figure 10 est un graphique des formes d'ondes des différentes grandeurs en pression P formant le jet pulsé représentées en fonction du temps « t » avec commande du front arrière de chute de pression à partir de l'aspiration.

Le procédé de base selon l'invention consiste, à l'aide de tous les moyens appropriés, mécaniques, électriques, électromécaniques, électromagnétiques ou autres, à générer sur commande à partir d'un premier conduit transportant un liquide stérile sous pression délivré par un générateur et d'un deuxième conduit pneumatique d'aspiration, un train impulsionnel d'un jet liquide stérile pulsé-aspiré et à le projeter par l'intermédiaire d'une pièce à main en vue d'applications chirurgicales ou médicales.

Plus particulièrement, on utilise pour chacun des conduits, de liquide d'une part et d'aspiration d'autre part, un moyen de fermeture-ouverture séquentiel du débit selon la même fréquence mais avec une durée d'ouverture pouvant être différente pour les deux fluides.

Selon le procédé de base de l'invention, le tir du jet liquide sous pression, c'est-à-dire l'ouverture du moyen de fermeture-ouverture du conduit de liquide, s'effectue pendant les intervalles de temps d'aspiration c'est-à-dire pendant la durée d'ouverture du moyen de fermeture-ouverture du conduit d'aspiration.

Le procédé est complété en ce que l'on réalise une neutralisation de l'aspiration par vide d'air juste après la fermeture du conduit d'aspiration, par exemple en mettant à l'air le conduit d'aspiration pour stopper l'aspiration.

Le procédé de base peut être mis en oeuvre par des moyens simples, comme des moyens électromécaniques à poussoirs ou autres agissant en pincement ou en écrasement sur un conduit souple véhiculant le liquide ou l'air aspiré ou par des composants hydrauliques de coupure tels que des obturateurs de débit ou des électrovannes, tous commandés par un circuit électronique de séquencement.

Il en est de même pour les moyens de mise à l'air périodique du conduit d'aspiration permettant de décoller le tissu à découper de l'extrémité aspirante de la pièce à main pour lesquels les moyens de coupure-ouverture seront pneumatiques et non plus hydrauliques.

On procède de préférence à une mise à l'air par le conduit d'aspiration par exemple par une dérivation de celui-ci périodiquement ouverte et fermée, mais toute autre façon de compenser la force de succion pour détendre le tissu est possible.

On expliquera maintenant le procédé à l'aide des différentes formes d'ondes.

Les principales phases caractéristiques de chaque période du train impulsionnel selon l'invention, repérées par les chiffres de 1 à 5 sur la figure 1, sont les suivantes :
. phase 1 : début de l'aspiration,
. phase 2 : tir du jet sous pression pendant une courte durée à l'intérieur de l'impulsion d'aspiration,
. phase 3 : poursuite de l'aspiration après le tir,
. phase 4 : mise à l'air pendant la coupure de l'aspiration,
. phase 5 : poursuite de la coupure de l'aspiration jusqu'à la prochaine période.

On remarque les caractéristiques générales suivantes à l'observation des figures. Le tir s'effectue avec un certain retard par rapport au début de l'aspiration. Le tir s'effectue de préférence dans la première moitié de la largeur d'impulsion de l'aspiration et s'arrête de préférence avant le début de la deuxième moitié. La mise à l'air a lieu après la coupure de l'aspiration et de préférence mais non obligatoirement juste après cette coupure.

On donne ci-après à titre d'exemple non limitatif les principales valeurs caractéristiques des paramètres du train impulsionnel de jet liquide sous pression pulsé-aspiré correspondant à celui représenté sur la figure 1.
. fréquence de récurrence (F_{R}) : 1 Hz
. largeur d'impulsion de tir : 100 ms
. largeur d'impulsion d'aspiration : 400 ms
. repos d'aspiration : 600 ms
. largeur d'impulsion de mise à l'air: 300 ms
. décalage entre la fin de l'impulsion d'aspiration et la mise à l'air : faible.

La mise à l'air est de courte durée et suit rapidement la fin de l'aspiration.

Les flancs de montée des impulsions sont représentés verticaux. Dans la pratique, la pente dépendra du type de dispositif de fermeture-ouverture de débit utilisé et notamment de son inertie.

D'autres formes d'ondes différentes sont possibles.

Ainsi, la fréquence de récurrence du régime impulsionnel et la largeur des impulsions sont modifiables. On peut ainsi effectuer des modifications doivent en fonction de l'application chirurgicale à savoir du type d'intervention, d'organe ou de tissu visé ainsi que de la profondeur d'intervention dans le corps humain.

L'appareil générant ce train commandé d'impulsions permettra de faire varier ces principaux paramètres dont les paramètres temporels. La fréquence de récurrence F_{R} peut par exemple être située dans une gamme comprise entre une fraction de Hertz et quelques Hertz, par exemple entre 0,1 et 10 Hz.

Il doit être noté que plus la largeur de l'impulsion du tir augmente, plus celle de l'aspiration doit augmenter pour pouvoir évacuer complètement le liquide et le ou les résidus.

La figure 2 montre des formes d'onde basées sur le même principe d'un jet liquide sous pression pulsé-aspiré, objet du procédé de base selon l'invention. On remarque que la durée du tir et la durée d'aspiration augmentent simultanément. En effet, plus la durée du tir est importante, plus l'aspiration doit être longue pour pouvoir évacuer tout le liquide projeté et les résidus solides. Corrélativement, les impulsions de commande ou les ouvertures correspondant à la mise à l'air se déplacent sur l'axe des temps pour débuter juste après la coupure de l'aspiration.

La figure 3 présente des formes d'onde sans mise à l'air. Cet exemple d'application est celui d'une pièce à main à manchon d'extrémité ouvert sur sa périphérie pour laquelle le relâchement du tissu après le tir s'effectue automatiquement après la coupure de l'aspiration.

On décrira maintenant la variante du procédé de base selon l'invention.

La figure 4 a pour but d'illustrer le problème résolu par la variante du procédé de base.

Lorsque la fréquence de récurrence F_{R} des impulsions périodiques de pression P formant la base de temps du jet pulsé augmente, il apparaît une déformation du front de descente des impulsions.

Cette déformation s'aggrave avec l'accroissement de la fréquence F_{R}. De plus, comme on peut le voir par l'augmentation de surface des zones hachurées, l'inclinaison et la forme de la rampe de descente se détériorent, la rampe devenant curviligne ou adoptant une allure exponentielle.

Cette rampe délimite avec le front arrière vertical idéal une zone d'inefficacité représentée hachurée, dont l'étendue augmente avec la fréquence de récurrence F_{R}. L'impulsion grossit en largeur par une prolongation arrière et la coupure de la pression n'est plus franche mais devient progressive.

La figure 4 permet de visualiser cet inconvénient.

L'inclinaison et la déformation de la rampe arrière provoquent une annulation de pression de plus en plus tardive dans le temps « t » tendant à rejoindre le prochain front de montée de pression. Les impulsions pourraient ainsi se rejoindre et le régime tendre vers un régime continu.

La déformation du front arrière et l'augmentation de largeur de l'impulsion de pression liquide diminuent l'efficacité du jet pulsé. L'effet d'impact répétitif s'amoindrit et ainsi la composante importante de l'efficacité du travail liée aux chocs périodiques s'annule progressivement.

Le présent perfectionnement a pour but de remédier à cet inconvénient en corrigeant la déformation du front arrière de l'impulsion de pression par une décompression commandée ou automatique du tube d'alimentation en liquide sous pression de la pièce à main. Cette décompression est synchronisée avec les impulsions de pression.

Cette variante au procédé de base procède de l'idée générale inventive qui consiste en une génération d'un train impulsionnel d'un jet liquide pulsé avec aspiration dans lequel le conduit d'alimentation du générateur de pression du liquide est mis en communication périodique avec un générateur de dépression ou avec l'air pour donner naissance à un régime impulsionnel d'éjection de liquide par une pièce à main selon une fréquence de récurrence F_{R}, dans lequel le front arrière de l'impulsion de pression du liquide est obtenu en coupant l'alimentation en liquide sous pression et en déchargeant le résiduel de pression subsistant ou qui subsisterait dans le conduit d'alimentation de la pièce à main en liquide sous pression par une mise en communication de ce conduit avec l'extérieur.

Selon le procédé de base, un générateur de liquide notamment stérile sous pression GHP est raccordé à une pièce à main 1 ou PAM par exemple chirurgicale, par un conduit tubulaire d'alimentation sous pression 2 à travers un organe de coupure-ouverture 3 commandé de façon périodique à une fréquence de récurrence F_{R}. Le liquide sous pression est, par exemple, généré et éjecté à une pression située entre 15 et 100 bars selon l'application visée.

Un circuit d'aspiration comprend un générateur de dépression GDP relié à la pièce à main 1 par un conduit indépendant d'aspiration 4 à travers un organe d'isolement-communication 5 commandé à la fréquence de récurrence F_{R} en synchronisme avec la commande en coupure-ouverture du conduit tubulaire d'alimentation 2 en liquide sous pression par l'organe de coupure-ouverture 3.

Un dispositif de mise à l'air (non représenté) est greffé sur le conduit d'aspiration. Il est commandé en synchronisme avec les autres commandes à la fréquence de récurrence F_{R}. Ce dispositif de mise à l'air a pour but de neutraliser l'aspiration et donc de détendre le tissu juste avant un nouveau tir.

Selon la présente variante, on prévoit une sortie adjacente sur le conduit tubulaire d'alimentation 2 en liquide sous pression, sous la forme d'une branche dérivée 6 permettant une mise en communication avec l'extérieur.

La fonction générale de cette branche dérivée 6 est celle de mettre périodiquement le conduit d'alimentation 2 en liquide sous pression en communication avec l'extérieur. Plus particulièrement cette fonction est celle de décompresser, c'est-à-dire d'évacuer rapidement le résiduel de pression, immédiatement avant ou après ou à la fermeture du conduit d'alimentation 2 en liquide sous pression.

Comme illustré par la figure 5, un des moyens de décompression possible est le raccordement à un générateur de dépression GDP, pouvant être le même que celui du circuit d'aspiration, et ceci à travers un dispositif interrupteur 7 assurant l'ouverture et la fermeture commandées de la communication avec ce générateur GDP et ceci en synchronisme avec la commande du jet sous pression.

Dans certains cas et selon la valeur de la fréquence de récurrence F_{R}, on pourra se contenter d'une communication avec l'atmosphère c'est-à-dire une mise à l'air périodique en synchronisme avec les impulsions de pression du tube d'alimentation 2 en liquide sous pression. Cette mise à l'air pourra être totale, partielle ou réduite par l'intermédiaire d'un dispositif commandé ou automatique de mise à l'air. Cette technique permettra ainsi dans ces cas de rétablir simplement la forme du front de descente de l'impulsion de pression, c'est-à-dire de rétablir une pente franche et raide.

Comme représenté sur la figure 6, il peut s'agir d'un organe de mise à l'air à échappement commandé MALC intégrant ou non l'interrupteur 7.

Comme exemple, on cite un clapet commandé ou une membrane perméable ou tout autre moyen analogue.

Comme représenté sur la figure 7, il peut s'agir aussi d'un organe de mise à l'air automatique, c'est-à-dire à échappement automatique. A titre d'exemple on cite un clapet taré avec effet de retard.

Ainsi, la pression résiduelle peut aussi jusqu'à une certaine fréquence être évacuée de façon simple et automatique par une sortie adjacente raccordée à un dispositif MALA à seuil de pression ou présentant un retard périodique constant à l'ouverture.

Bien entendu, il faut comprendre le terme extérieur dans son sens le plus général, c'est-à-dire un espace ou un volume extérieur au conduit d'alimentation 2. En effet, il peut s'agir aussi d'un volume tampon, d'un volume fermé extensible, d'une membrane souple...

On peut également commander cette coupure par la mise en communication du conduit de pression 2 avec le générateur de dépression GDP. Il suffit de commander l'ouverture de la communication avec l'extérieur par la branche dérivée 6 du conduit d'alimentation 2 en liquide sous pression peu avant la coupure du liquide sous pression.

Cette façon de procéder que l'on décrira maintenant est illustrée par la figure 10.

Selon la caractéristique principale du procédé de base, le tir s'effectue pendant la phase d'aspiration, c'est-à-dire que l'impulsion de pression est déclenchée et coupée dans l'intervalle de temps correspondant à un créneau de dépression.

On a représenté sur la figure 10 la commande de la mise à l'air de l'aspiration qui s'effectue juste après la coupure de l'aspiration pour favoriser le décollement et la relaxation des tissus.

La deuxième partie de la figure 10 illustre la commande de la fin du tir par l'aspiration.

L'impulsion de pression du tir à l'extrémité de la pièce à main sans correction est celle représentée sur la troisième courbe laissant apparaître la zone d'inefficacité représentée hachurée.

Selon cette variante, on commande la coupure de l'impulsion de tir par la dépression avant sa coupure normale par l'organe 3 d'ouverture-fermeture.

Plus particulièrement, selon la présente variante, l'impulsion de pression débutant au temps t1 est coupée en avance au temps t2 par une ouverture rapide commandée de la communication avec l'extérieur et notamment avec l'aspiration. On calibre ainsi l'impulsion de tir à une durée T en réalisant une coupure raide et franche comme il apparaît sur la dernière forme d'onde représentée sur cette figure 10.

La durée de l'impulsion de dépression ou d'aspiration du conduit de pression de tir qui déclenche la coupure (4ème courbe) est suffisante pour réaliser la décharge totale de la pression résiduelle. Elle reste cependant faible.

L'impulsion de dépression ou d'aspiration a pour but de créer un appel de liquide et donc une décharge avant ou juste avant la coupure de la pression du liquide, neutralisant ainsi la rémanence de pression et garantissant une tombée brusque de la pression.

La présente invention est mise en oeuvre par une source de liquide sous pression et par un appareil de séquencement c'est-à-dire d'arrêt et de démarrage brutal du jet liquide sous pression alimentant une pièce à main ou un cathéter.

La formation des impulsions peut provenir des actions mécaniques répétitives par exemple sur un conduit souple.

En mode_ de fonctionnement normal c'est-à-dire courant, le train d'impulsions déclenché par l'opérateur se poursuit à la fréquence de récurrence F_{R} jusqu'à la commande manuelle d'arrêt ordonnée par l'opérateur.

Dans différents cas d'application, il est souhaitable de limiter autrement et automatiquement le nombre de jets élémentaires liquides sous pression. On y procédera par un programme adapté de commande et de comptage/décomptage. L'opérateur par sa commande digitale sur la pièce à main déclenchera un nombre limité de jets élémentaires liquides sous pression se succédant à la fréquence de récurrence F_{R}. Cette séquence s'arrêtera automatiquement lorsque le nombre d'impulsions de tir programmé sera atteint.

Le nombre de jets élémentaires liquides sous pression n'est limité que par des considérations d'ordre pratique liées à l'intervention et par les possibilités du matériel et du logiciel.

On peut aussi envisager de ne programmer qu'un seul tir d'un jet élémentaire liquide sous pression. Dans ce cas l'opérateur ne déclenchera qu'un seul tir par sa commande manuelle. Le tir suivant devra être déclenché par une nouvelle commande.

## Revendications

1. Procédé de génération sur commande d'un train impulsionnel d'un jet pulsé de liquide pour une pièce à main en vue notamment d'un travail chirurgical de dissection, le travail chirurgical s'effectuant par des trains d'impulsions périodiques d'un liquide sous pression formés chacun par une succession de commandes périodiques en ouverture et en fermeture du liquide sous pression selon une fréquence de récurrence F_{R} pour former des impulsions de pression constituant des tirs de jets élémentaires de liquide sous pression mettant en oeuvre une voie de liquide sous pression provenant d'un générateur de liquide sous pression GHP et un train d'impulsions périodiques de dépression mettant en oeuvre une voie d'aspiration reliée à une source de dépression GDP, les impulsions de pression et les impulsions de dépression présentant la même fréquence de récurrence F_{R}, **caractérisé en ce que** :
. les impulsions de pression sont inférieures en durée aux impulsions de dépression,
. les impulsions de pression sont situées temporellement à l'intérieur des impulsions de dépression et s'effectuent avec un certain retard par rapport an début des impulsions de dépression, créant ainsi un jet pulsé-aspiré.

2. Procédé selon la revendication 1 **caractérisé en ce que** le liquide sous pression est généré et expulsé sous haute pression.

3. Procédé selon la revendication précédente **caractérisé en ce que** la haute pression est située entre 15 et 100 bars.

4. Procédé selon l'une quelconque des revendications précédentes **caractérisé en ce que** l'on commande le tir périodique du jet sous pression dans la première moitié de la durée périodique d'application de l'aspiration.

5. Procédé selon la revendication précédente **caractérisé en ce que** la durée périodique de tir du jet sous pression se termine avant la première moitié de l'impulsion périodique d'aspiration.

6. Procédé selon l'une quelconque des revendications précédentes **caractérisé en ce que** l'aspiration est suivie après sa coupure par une compensation de l'effet de succion pour détendre le tissu.

7. Procédé selon la revendication précédente **caractérisé en ce que** la compensation de l'effet de succion est une mise à l'air pendant la coupure de l'aspiration.

8. Procédé selon la revendication précédente **caractérisé en ce que** l'on procède à la mise à l'air par le conduit d'aspiration.

9. Procédé selon l'une quelconque des revendications précédentes **caractérisé en ce que** la fréquence de récurrence F_{R} est modifiable.

10. Procédé selon l'une quelconque des revendications précédentes **caractérisé en ce que** la fréquence de récurrence F_{R} est située dans une gamme comprise entre une fraction de Hertz et quelques Hertz.

11. Procédé selon la revendication précédente **caractérisé en ce que** la fréquence de récurrence F_{R} est comprise entre 0,1 et 10 Hz.

12. Procédé selon la revendication précédente **caractérisé en ce que** la fréquence de récurrence F_{R} est de 1 Hz.

13. Procédé selon l'une quelconque des revendications précédentes **caractérisé en ce que** le front arrière de l'impulsion de pression P est obtenu en déchargeant, au voisinage du moment de la coupure de l'alimentation en liquide sous pression, le conduit d'alimentation (2) en liquide sous pression de la pièce à main PAM par une mise en communication avec l'extérieur de ce conduit (2).

14. Procédé selon la revendication 13 **caractérisé en ce que** la mise en communication avec l'extérieur est une mise en communication avec un générateur de dépression GDP.

15. Procédé selon la revendication 13, **caractérisé en ce que** la mise en communication avec l'extérieur est une mise à l'air.

16. Procédé selon la revendication 13 **caractérisé en ce que** la mise en communication avec l'extérieur qui s'effectue au voisinage du moment de la coupure est une décharge dans un volume tampon.

17. Procédé selon la revendication 13 **caractérisé en ce que** la mise en communication avec l'extérieur qui s'effectue au voisinage du moment de la coupure est une décharge dans un volume fermé extensible.

18. Procédé selon l'une quelconque des revendications 13 à 17 **caractérisé en ce que** la mise en communication avec l'extérieur s'effectue un peu avant la coupure.

19. Procédé selon l'une quelconque des revendications 13 à 17 **caractérisé en ce que** la mise en communication avec l'extérieur s'effectue au moment de la coupure.

20. Procédé selon l'une quelconque des revendications 13 à 17 **caractérisé en ce que** la mise en communication avec l'extérieur s'effectue un peu après la coupure.

21. Procédé selon l'une quelconque des revendications 13 à 20 **caractérisé en ce que** la durée de l'aspiration déchargeant le tube d'alimentation (2) est faible par rapport à la durée entre le front arrière de l'impulsion de pression et le front avant de la prochaine impulsion de pression.

22. Procédé selon les revendications 13 et 14 **caractérisé en ce que** l'on commande la coupure de l'impulsion de pression de liquide par la commande de la mise en communication avec le générateur de dépression GDP.

23. Procédé selon l'une quelconque des revendications précédentes **caractérisé en ce que** l'on commande un nombre défini de jets élémentaires de liquide sous pression.

24. Procédé selon l'une quelconque des revendications précédentes **caractérisé en ce que** l'on commande un seul jet élémentaire de liquide sous pression à la fois.

25. Circuit pour la mise en oeuvre du procédé selon l'une quelconque des revendications précédentes composé d'une pièce à main, d'un générateur de pression d'un liquide GHP, d'une liaison tubulaire par un conduit (2) pour l'alimentation en liquide sous pression de la pièce à main (1), d'un générateur de dépression GDP relié à la pièce à main par un conduit séparé (4), d'un organe (3) de coupure-ouverture du passage du liquide sous pression et d'un organe d'isolement et de mise en communication (5) du conduit (4) avec le générateur de dépression GDP, **caractérisé en ce que** le tube d'alimentation (2) de la pièce à main (1) en liquide sous pression présente une branche dérivée (6) pour la liaison avec l'extérieur.

26. Circuit selon la revendication précédente **caractérisé en ce que** la branche dérivée (6) est raccordée à un générateur de dépression GDP à travers un organe d'isolement-communication (7) commandé en synchronisme avec la commande en ouverture-fermeture du conduit (2) d'alimentation de la pièce à main (1) en liquide sous pression.

27. Circuit selon la revendication 25 **caractérisé en ce que** la branche dérivée (6) est raccordée à un dispositif de mise à l'air commandé MALC à travers un organe d'isolement-communication (7) commandé en synchronisme avec la commande en ouverture-fermeture du conduit (2) d'alimentation de la pièce à main (1) en liquide sous pression.

28. Circuit selon la revendication 25 **caractérisé en ce que** la branche dérivée (6) est raccordée à un dispositif de mise à l'air automatique MALA.

29. Circuit selon la revendication 26 **caractérisé en ce que** le générateur de dépression GDP est unique.

## Patentansprüche

1. Verfahren zur gesteuerten Erzeugung einer Impulsfolge eines gepulsten Flüssigkeitsstrahls für ein Handstück, insbesondere zur chirurgischen Sezierungsarbeit, wobei die chirurgische Arbeit mittels Folgen periodischer Pulse einer unter Druck stehenden Flüssigkeit durchgeführt wird, die jeweils durch eine periodische Befehlsabfolge zum Freigeben und Sperren der unter Druck stehenden Flüssigkeit mit einer Wiederholungsfrequenz F_{R} gebildet werden, um Druckpulse zu erzeugen, die Schüsse aus Elementarstrahlen aus unter Druck stehender Flüssigkeit bilden, wobei ein Weg für unter Druck stehende Flüssigkeit Verwendung findet, die aus einer Erzeugungseinrichtung GHP für unter Druck stehende Flüssigkeit stammt, und um eine Folge periodischer Druckabfall-Pulse zu erzeugen, wobei ein mit einer Druckabfall-Quelle GDP verbundener Absaugweg Verwendung findet, wobei die Druckpulse und die Druckabfall-Pulse dieselbe Wiederholungsfrequenz F_{R} aufweisen, **dadurch gekennzeichnet, dass**:
- die Druckpulse eine kürzere Dauer als die Druckabfall-Pulse aufweisen,
- die Druckpulse zeitlich innerhalb der Druckabfall-Pulse liegen und mit einer bestimmten Verzögerung bezogen auf den Anfang der Druckabfall-Pulse erfolgen, so dass ein gepulst-abgesaugter Strahl erzeugt wird.

2. Verfahren nach Anspruch 1, **dadurch gekennzeichnet, dass** die unter Druck stehende Flüssigkeit unter hohem Druck erzeugt und ausgestoßen wird.

3. Verfahren nach Anspruch 2, **dadurch gekennzeichnet, dass** der hohe Druck zwischen 15 und 100 bar beträgt.

4. Verfahren nach einem der Ansprüche 1 bis 3, **dadurch gekennzeichnet, dass** der periodische Schuss des unter Druck stehenden Strahls in der ersten Hälfte der periodischen Dauer der Anwendung der Absaugung gesteuert wird.

5. Verfahren nach Anspruch 4, **dadurch gekennzeichnet, dass** die periodische Schussdauer des unter Druck stehenden Strahls vor der ersten Hälfte des periodischen Absaugpulses endet.

6. Verfahren nach einem der Ansprüche 1 bis 5, **dadurch gekennzeichnet, dass** auf die Absaugung nach deren Abschaltung eine Kompensierung des Saugeffekts folgt, um das Gewebe zu entlasten.

7. Verfahren nach Anspruch 6, **dadurch gekennzeichnet, dass** die Kompensierung des Saugeffekts ein Belüften während der Abschaltung der Absaugung ist.

8. Verfahren nach Anspruch 7, **dadurch gekennzeichnet, dass** das Belüften über die Absaugleitung erfolgt.

9. Verfahren nach einem der Ansprüche 1 bis 8, **dadurch gekennzeichnet, dass** die Wiederholungsfrequenz F_{R} veränderbar ist.

10. Verfahren nach einem der Ansprüche 1 bis 9, **dadurch gekennzeichnet, dass** die Wiederholungsfrequenz F_{R} in einem Bereich zwischen einem Bruchteil eines Hertz und einigen Hertz liegt.

11. Verfahren nach Anspruch 10, **dadurch gekennzeichnet, dass** die Wiederholungsfrequenz F_{R} zwischen 0,1 und 10 Hz beträgt.

12. Verfahren nach Anspruch 11, **dadurch gekennzeichnet, dass** die Wiederholungsfrequenz F_{R} 1 Hz beträgt.

13. Verfahren nach einem der Ansprüche 1 bis 12, **dadurch gekennzeichnet, dass** die hintere Flanke des Druckpulses P **dadurch** erzeugt wird, dass in der Nähe des Zeitpunkts der Abschaltung der Versorgung des Handstücks PAM mit unter Druck stehender Flüssigkeit die Leitung (2) zur Versorgung des Handstücks RAM mit unter Druck stehender Flüssigkeit durch ein Verbinden der Leitung (2) mit einem äußeren Raum entleert wird.

14. Verfahren nach Anspruch 13, **dadurch gekennzeichnet, dass** das Verbinden mit dem äußeren Raum ein Verbinden mit einem Druckabfall-Erzeuger GDP ist.

15. Verfahren nach Anspruch 13, **dadurch gekennzeichnet, dass** das Verbinden mit dem äußeren Raum ein Belüften ist.

16. Verfahren nach Anspruch 13, **dadurch gekennzeichnet, dass** das Verbinden mit dem äußeren Raum, das in der Nähe des Zeitpunkts der Abschaltung erfolgt, ein Entleeren in ein Puffervolumen ist.

17. Verfahren nach Anspruch 13, **dadurch gekennzeichnet, dass** das Verbinden mit dem äußeren Raum, das in der Nähe des Zeitpunkts der Abschaltung erfolgt, ein Entleeren in ein geschlossenes ausdehnbares Volumen ist.

18. Verfahren nach einem der Ansprüche 13 bis 17, **dadurch gekennzeichnet, dass** das Verbinden mit dem äußeren Raum etwas vor der Abschaltung erfolgt.

19. Verfahren nach einem der Ansprüche 13 bis 17, **dadurch gekennzeichnet, dass** das Verbinden mit dem äußeren Raum im Zeitpunkt der Abschaltung erfolgt.

20. Verfahren nach einem der Ansprüche 13 bis 17, **dadurch gekennzeichnet, dass** das Verbinden mit dem äußeren Raum etwas nach der Abschaltung erfolgt.

21. Verfahren nach einem der Ansprüche 13 bis 20, **dadurch gekennzeichnet, dass** die Dauer der Absaugung zum Entleeren des Versorgungsschlauchs (2) gering ist gegenüber der Dauer zwischen der hinteren Flanke des Druckpulses und der vorderen Flanke des nächsten Druckpulses.

22. Verfahren nach den Ansprüchen 13 und 14, **dadurch gekennzeichnet, dass** das Abschalten des Flüssigkeits-Druckpulses durch die Steuerung zum Verbinden mit dem Druckabfall-Erzeuger GDP gesteuert wird.

23. Verfahren nach einem der Ansprüche 1 bis 22, **dadurch gekennzeichnet, dass** eine definierte Anzahl von Elementarstrahlen mit unter Druck stehender Flüssigkeit gesteuert wird.

24. Verfahren nach einem der Ansprüche 1 bis 23, **dadurch gekennzeichnet, dass** jeweils ein einzelner Elementarstrahl mit unter Druck stehender Flüssigkeit gesteuert wird.

25. Schaltung zum Durchführen des Verfahrens nach einem der vorangehenden Ansprüche, mit einem Handstück, einem Druckerzeuger GHP für eine Flüssigkeit, einer röhrenförmigen Verbindung über eine Leitung (2) zur Versorgung des Handstücks (1) mit einer unter Druck stehenden Flüssigkeit, einem Druckabfall-Erzeuger GDP, der mit dem Handstück über eine getrennte Leitung (4) verbunden ist, einem Organ (3) zum Verschließen und Öffnen des Durchgangs für die unter Druck stehende Flüssigkeit und einem Organ (5) zum Isolieren und Verbinden der Leitung (4) von bzw. mit dem Druckabfall-Erzeuger GDP, **dadurch gekennzeichnet, dass** die Leitung (2) zum Versorgen des Handstücks (1) mit unter Druck stehender Flüssigkeit einen Nebenzweig (6) für die Verbindung mit der Umgebung aufweist.

26. Schaltung nach Anspruch 25, **dadurch gekennzeichnet, dass** der Nebenzweig (6) über ein Isolier- und Verbindeorgan (7) an einen Druckabfall-Erzeuger GDP angeschlossen ist, das synchron mit der Öffnungs- und Verschlusssteuerung der Leitung (2) zur Versorgung des Handstücks (1) mit unter Druck stehender Flüssigkeit gesteuert ist.

27. Schaltung nach Anspruch 25, **dadurch gekennzeichnet, dass** der Nebenzweig (6) über ein Isolier- und Verbindeorgan (7) an eine gesteuerte Belüftungseinrichtung MALC angeschlossen ist, das synchron mit der Öffnungs- und Verschlusssteuerung der Leitung (2) zur Versorgung des Handstücks (1) mit unter Druck stehender Flüssigkeit gesteuert ist.

28. Schaltung nach Anspruch 25, **dadurch gekennzeichnet, dass** der Nebenzweig (6) an eine automatische Belüftungseinrichtung MALA angeschlossen ist.

29. Schaltung nach Anspruch 26, **dadurch gekennzeichnet, dass** ein einzelner Druckabfall-Generator DGDP vorgesehen ist.

## Claims

1. Method for generating on command a pulse train of a pulsed jet of liquid for a handpiece with the aim in particular of surgical dissection work, the surgical work being performed by periodic pulse trains of a pressurised liquid each formed by a succession of periodic opening and closing commands for the pressurised liquid according to a recurrence frequency F_{R} to form pressure pulses constituting elementary jet streams of pressurised liquid using a pressurised liquid generator GHP and a train of periodic vacuum pulses using a suction channel linked to a vacuum source GDP, the pressure pulses and vacuum pulses having the same recurrence frequency F_{R},
**characterised in that**
- the pressure pulses are shorter in duration than the vacuum pulses,
- the pressure pulses are temporally located within the vacuum pulses and are performed with a particular delay in relation to the start of the vacuum pulses, thus creating a pulse-suction jet.

2. Method according to claim 1, **characterised in that** the pressurised liquid is generated and expelled under high pressure.

3. Method according to the previous claim, **characterised in that** the high pressure is between 15 and 100 bar.

4. Method according to any of the previous claims,
**characterised in that** the periodic pulse of the pressurised jet is commanded in the first half of the periodic duration of suction application.

5. Method according to the previous claim, **characterised in that** the periodic duration of the pressurised jet pulse is completed before the first half of the periodic suction pulse.

6. Method according to any of the previous claims,
**characterised in that** after its interruption, the suction is followed by compensation for the suction effect to relax the tissue.

7. Method according to the previous claim, **characterised in that** the compensation for the suction effect is an exposure to air during the suction interruption.

8. Method according to the previous claim, **characterised in that** the exposure to air is achieved through the suction pipe.

9. Method according to any of the previous claims,
**characterised in that** the recurrence frequency F_{R} is modifiable.

10. Method according to any of the previous claims,
**characterised in that** the recurrence frequency F_{R} lies in a range between a fraction of a Hertz and a few Hertz.

11. Method according to the previous claim, **characterised in that** the recurrence frequency F_{R} is between 0.1 and 10 Hz.

12. Method according to the previous claim, **characterised in that** the recurrence frequency F_{R} is 1 Hz.

13. Method according to any of the previous claims,
**characterised in that** the trailing flank of the pressure pulse P is obtained by discharging, close to the time of interruption of the pressurised liquid supply, the pressurised liquid supply pipe (2) of the handpiece PAM by establishing communication with the exterior of this pipe (2).

14. Method according to claim 13 **characterised in that** the establishment of communication with the exterior is the establishment of communication with a vacuum generator GDP.

15. Method according to claim 13, **characterised in that** the establishment of communication with the exterior is exposure to air.

16. Method according to claim 13, **characterised in that** the establishment of communication with the exterior close to the time of interruption is a discharge into a buffer volume.

17. Method according to claim 13, **characterised in that** the establishment of communication with the exterior close to the time of interruption is a discharge into an expandable closed volume.

18. Method according to any of claims 13 to 17, **characterised in that** communication with the exterior is established slightly before interruption.

19. Method according to any of claims 13 to 17, **characterised in that** communication with the exterior is established at the time of interruption.

20. Method according to any of claims 13 to 17, **characterised in that** communication with the exterior is established shortly after interruption.

21. Method according to any of claims 13 to 20, **characterised in that** the duration of suction discharging the supply pipe (2) is short in relation to the duration between the trailing flank of one pressure pulse and the leading flank of the next pressure pulse.

22. Method according to any of claims 13 and 14,
**characterised in that** the interruption of the pressure pulse of the liquid is controlled by the command for establishing communication with the vacuum generator GDP.

23. Method according to any of the previous claims,
**characterised in that** a defined number of elementary jets of pressurised liquid are controlled.

24. Method according to any of the previous claims,
**characterised in that** a single elementary jet of pressurised liquid is controlled at a time.

25. Circuit for implementation of the method according to any of the previous claims, comprising a handpiece, a liquid pressure generator GHP, a tubular link through a pipe (2) to supply pressurised liquid to the handpiece (1), a vacuum generator GDP linked to the handpiece by a separate pipe (4), an element (3) for interruption-opening of the passage of pressurised liquid and an element for isolation and communication (5) of the pipe (4) with the vacuum generator GDP, **characterised in that** the pipe (2) supplying the handpiece (1) with pressurised liquid has a branch (6) for the linking to the exterior.

26. Circuit according to the previous claim, **characterised in that** the branch (6) is connected to a vacuum generator GDP through an isolation-communication element (7) controlled in synchrony with the interrupting-opening command of the pipe (2) supplying the handpiece (1) with pressurised liquid.

27. Circuit according to claim 25, **characterised in that** the branch (6) is connected to a controlled air exposure device MALC through an isolation-communication element (7) controlled in synchrony with the opening-closing command of the pipe (2) for supplying the handpiece (1) with pressurised liquid.

28. Circuit according to claim 25, **characterised in that** the branch (6) is connected to an automatic air exposure device MALA.

29. Circuit according to claim 26, **characterised in that** the vacuum generator GDP is a single generator.
